(19) Europäisches Patentamt
European Patent Office
Office européen des brevets

(11) Publication number: 0 072 121
A1

# (12) EUROPEAN PATENT APPLICATION

(21) Application number: **82303859.1**

(22) Date of filing: **22.07.82**

(51) Int. Cl.³: **C 07 D 487/04**, A 61 K 31/40
// (C07D487/04, 209/00, 205/00)

(30) Priority: **08.08.81 GB 8124312**
**09.11.81 GB 8133768**

(43) Date of publication of application: **16.02.83**
**Bulletin 83/7**

(84) Designated Contracting States: **BE CH DE FR GB IT LI NL SE**

(71) Applicant: **BEECHAM GROUP PLC, Beecham House Great West Road, Brentford Middlesex (GB)**

(72) Inventor: **Coulton, Steve, 6 Badgers Close, Horsham West Sussex (GB)**
Inventor: **Corbett, David Francis, 72E Holmesdale Road, Reigate Surrey (GB)**
Inventor: **Southgate, Robert, 7 Tilletts Lane, Warnham West Sussex (GB)**

(74) Representative: **Hesketh, Alan, Dr. et al, European Patent Attorney Beecham Pharmaceuticals Great Burgh Yew Tree Bottom Road, Epsom, Surrey, KT18 5XQ (GB)**

(54) **Beta-lactam antibiotics.**

(57) An amidine derivative of an amine of formula (II):

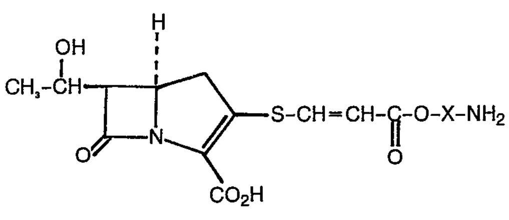

(II)

or a pharmaceutically acceptable salt or in vivo hydrolysable ester thereof where X represents a $C_{1-6}$ alkanediyl moiety.

Processes for the preparation and compositions containing such compound.

EP 0 072 121 A1

# β-LACTAM ANTIBIOTICS

This invention relates to novel 7-oxo-1-azabicyclo[3.2.0] heptene derivatives and in particular to such derivatives with C-3 carboxyvinylthio substituents. This invention further relates to processes for their preparation and to compositions containing them. These derivatives are of use in the treatment of bacterial infection.

European Patent Application Publication No. 0024832 discloses *inter alia* compounds of the formula (I):

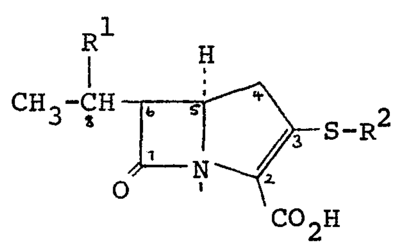

(I)

and pharmaceutically acceptable salts and *in vivo* hydrolysable esters thereof wherein $R^1$ is a hydrogen atom or a group selected from OH, $OSO_3H$ or a pharmaceutically acceptable salt or a methyl or ethyl ester thereof, $OR^3$, $SR^4$, $OCOR^3$, $OCO_2R^4$ or $OCONHR^4$ where $R^3$ is a $C_{1-4}$ alkyl group or a benzyl group and $R^4$ is a $C_{1-4}$ alkyl, benzyl, phenyl or p-nitrobenzyl group; and $R^2$ is $C_{1-4}$alkyl, $C_{1-4}$alkyl substituted by a phenyl group

optionally substituted by a fluorine, chlorine or bromine atom or a methoxy, nitro, amino, acetamido or p-nitrobenzylcarbonylamino group; a $C_{2-4}$ alkyl group substituted on other than the α-carbon atom by an amino, p-nitrobenzyloxycarbonylamino, hydroxy, p-nitrobenzyloxycarbonyloxy, methoxy, acetoxy or $C_{1-4}$ alkyloxycarbonyl group; a $C_{1-4}$acyl group or a benzoyl, phenylacetyl or phenoxyacetyl group; or a $CH=CHCO_2H$ group or a pharmaceutically acceptable salt or ester thereof.

We have now found that compounds within formula (I) having $R^1$ equal to hydroxy and $R^2$ being an amidino $C_{1-6}$ alkyl ester of $-SCH=CHCO_2H$ show superior efficacy for example antibacterial activity and/or stability than related compounds specifically disclosed in the aforementioned European Patent Application.

The present invention relates to amidine derivatives of an amine compound of the formula (II):

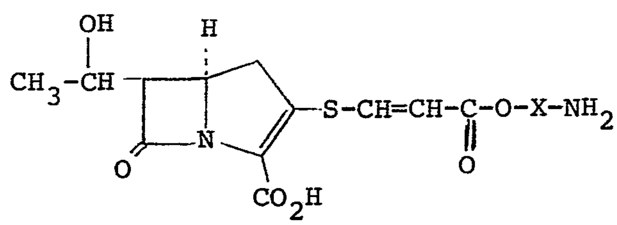

(II)

or a pharmaceutically acceptable salt or *in vivo* hydrolysable ester thereof, wherein X is a $C_{1-6}$ alkanediyl moiety.

Accordingly the present invention provides the compounds of the formula (III):

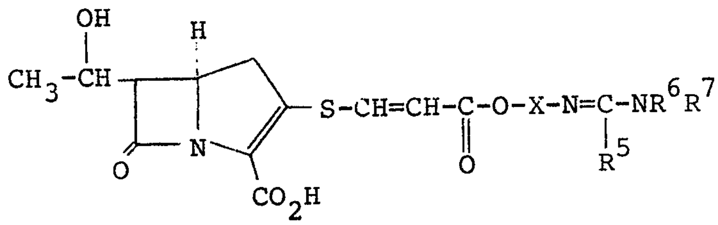

(III)

or a pharmaceutically acceptable salt or in vivo hydrolysable ester thereof, wherein X is a $C_{1-6}$alkanediyl moiety, and $R^5$, $R^6$ and $R^7$ are independently selected from hydrogen, a heterocyclic group, a hydrocarbon group up to 18 carbon atoms; or $R^6$ and $R^7$ together with the nitrogen atom to which they are attached form a $C_{3-10}$ cycloalkyl ring; any of the above $R^5$, $R^6$ and $R^7$ groups being optionally substituted. Suitably $R^5$, $R^6$ and $R^7$ are selected from hydrogen, $C_{1-10}$alkyl, $C_{3-10}$cycloalkyl, aryl $C_{1-6}$alkyl, $C_{3-10}$cycloalkyl $C_{1-6}$alkyl, aryl or heterocyclyl having 5 - 6 ring atoms, up to 4 of which are selected from O, N and S; or $R^6$ and $R^7$ are joined so as to form a $C_{3-10}$ cycloalkyl ring; any of the above $R^5$, $R^6$ and $R^7$ groups being optionally substituted.

When used herein alkanediyl means a divalent alkylene moiety. Suitable optional substituents for the groups $R^5$, $R^6$ and $R^7$ include $C_{1-6}$ alkyl, amino, hydroxy, $C_{1-6}$ alkoxy, $C_{1-6}$ alkylthio, mercapto, chloro, bromo, fluoro, carboxy and $C_{1-6}$alkanoyloxy.

Suitably X is a polymethylene moiety for example methylene, ethylene, trimethylene, tetramethylene or pentamethylene. Of these ethylene is preferred.

Suitably $R^5$ is $C_{1-6}$alkyl such as methyl, ethyl or propyl, aryl ($C_{1-6}$) alkyl such as benzyl or phenethyl, $C_{3-7}$ cycloalkyl such as cyclohexyl, aryl such as phenyl, heterocyclyl such as pyridyl or pyrimidyl or suitably R is hydrogen.

Suitably $R^6$ and $R^7$ are independently selected from hydrogen or $C_{1-6}$alkyl such as methyl, ethyl or propyl, aryl

$(C_{1-6})$ alkyl such as benzyl or phenethyl, $C_{3-7}$cycloalkyl such as cyclohexyl, or aryl such as phenyl. In an alternative aspect $R^6$ and $R^7$ together with the nitrogen atom to which they are attached form a $C_{3-10}$ cycloalkyl ring for example cyclohexyl or cyclopentyl.

A preferred group of compounds of this invention is that of the formula (IV):

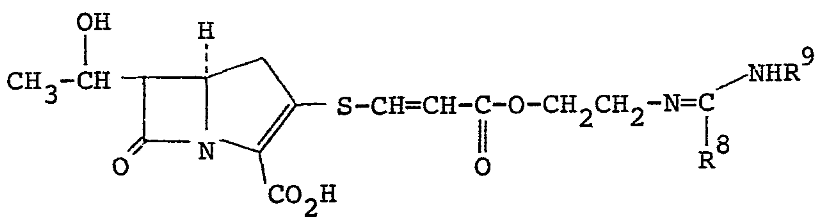

(IV)

or a pharmaceutically acceptable salt or in vivo hydrolysable ester thereof, wherein $R^8$ is hydrogen or $C_{1-4}$ alkyl and $R^9$ is hydrogen or $C_{1-4}$ alkyl. Most favourably $R^8$ is hydrogen or methyl and $R^9$ is hydrogen.

The compounds of the formulae (III) - (IV) may have R or S stereochemistry at the C-8 position (that is the α-carbon atom of the C-6 substituent) or may be in the form of mixtures thereof.

The compounds of the formulae (III) - (IV) may be provided with a cis configuration of the C-5 and C-6 protons, or they may be provided with a trans configuration of the C-5 and C-6 protons. Alternatively they are provided as a mixture of the cis and trans forms. In the compounds of the formulae (III) - (IV) the double bond present in the C-3 thio substituent

may be present in either the E configuration or the Z configuration, or the compounds may be provided as mixtures thereof.

It is to be realised that where $R^7$ is hydrogen the compounds of the formula (III) may be represented by the structure (V):

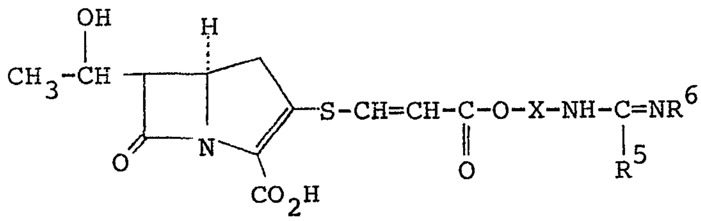

(V)

The compounds of the formulae (III) - (IV) most suitably may be presented in the form of the carboxylic free acid at the C-2 position, said compounds preferably being in zwitterionic form.

Examples of suitable pharmaceutically acceptable in vivo hydrolysable ester groups include those which break down readily in the human body to leave the parent acid or its salt, for example acyloxyalkyl groups such as acetoxymethyl, pivaloyloxymethyl, α-acetoxyethyl and α-pivaloyloxyethyl groups; alkoxycarbonyloxyalkyl groups, such as ethoxycarbonyloxymethyl and α-ethoxycarbonyloxyethyl; dialkylaminoalkyl groups such as dimethylaminomethyl, dimethylaminoethyl, diethylaminomethyl or diethylaminoethyl; lactone groups such as phthalidyl and dimethoxyphthalidyl; and esters formed from the residue of a second β-lactam antibiotic.

Suitable pharmacuetically acceptable salts of the carboxy group of the compounds of this invention include metal salts eg aluminium, alkali metal salts such as sodium or potassium, alkaline earth metal salts such as calcium or magnesium and ammonium or substituted ammonium salts, for example those with lower alkylamines such as triethylamine, hydroxy-lower alkylamines such as 2-hydroxyethylamine, bis-(2-hydroxyethyl)-amine or tris-(2-hydroxyethyl)-amine, cycloalkylamines such as dicyclohexylamine, or with procaine, dibenzylamine, N,N-dibenzylethylene-diamine, l-ephenamine, N-ethylpiperidine, N-benzyl-β-phenethylamine, dehydroabietylamine, N,N'-bisdehydro-abietylamine, ethylenediamine, or bases of the pyridine type such as pyridine, collidine or quinoline, or other amines which have been used to form salts with known penicillins and cephalosporins.

The compounds of the formulae (III) - (IV), may be employed in the treatment of bacterial infection. Thus the present invention also provides a pharmaceutical composition which comprises a compound of the formula (III) - (IV) and a pharmaceutically acceptable carrier.

The compositions of this invention may be prepared by conventional methods of preparing antibiotic compositions and in conventional manner may be adapted for oral, topical or parenteral administration.

Aptly, the compositions of this invention are in the form of a unit-dose composition adapted for oral administration.

Alternatively the compositions of this invention are in the form of a unit dose composition adapted for administration by injection.

Unit-dose forms according to this invention will normally contain from 50 to 500 mgs of a compound of this invention, for example about 62.5, 100, 125, 150, 200, 250 or 300 mgs. Such compositions may be administered from 1 to 6 times a day or more conveniently 2, 3 or 4 times a day so that the total daily dose for a 70 kg adult is about 200 to 2000 mg, for example about 400, 600, 750, 1000 or 1500 mg.

The compositions of this invention may be used to treat bacterial infection in mammals including humans for example infections of the respiratory tract, urinary tract or soft tissues, or mastitis in cattle. Such compositions may be administered to susceptible gram-positive or gram-negative bacteria such as strains of *Staphylococcus aureus*, *Klebsiella aerogenes* and *Escherichia coli*.

The carriers used in the compositions of this invention may include diluents, binders, disintegrants, lubricants, colours, flavouring agents and preservatives in conventional manner. Thus suitable agents include lactose, starch, sucrose, calcium phosphate, sorbitol, polyvinylpyrrolidone, acacia, gelatin, tragacanth or the like, potato starch or polyvinylpolypyrrolidone, magnesium stearate and sodium lauryl sulphate.

Orally administrable forms of the compositions of this invention are most suitably in the form of unit-dose units such as tablets or capsules.

The present invention also provides synergistic pharmaceutical compositions which comprise a pharmaceutical composition as hereinbefore described which also contains a penicillin or a cephalosporin.

Suitable penicillins for inclusion in the compositions of this invention include benzyl penicillin, phenoxymethylpenicillin, ampicillin or a pro-drug therefore, amoxycillin or a pro-drug therefor, carbenicillin of a pro-drug therefor, ticarcillin or a pro-drug therefore, suncillin, sulbenicillin, azlocillin and mezlocillin.

Particularly suitable penicillins for inclusion in orallyadministrable compositions of this invention include ampicillin and its orally administrable pro-drugs, amoxycillin and its orally administrable pro-drugs and orally administrable pro-drugs of carbenicillin. Thus particularly suitable penicillins include ampicillin anhydrate, ampicillin trihydrate, sodium ampicillin, talampicillin hydrochloride, pivampicillin hydrochloride, bacampicillin hydrochloride; amoxycillin trihydrate, sodium amoxycillin; and the sodium salts of the phenyl and 5-indanyl α-esters of carbenicillin.

A preferred penicillin for inclusion in the orally administrable compositions of this invention is amoxycillin trihydrate. A further preferred penicillin for inclusion in the orally administrable compositions of this invention is ampicillin trihydrate.

Particularly suitable penicillins for inclusion

in injectably administrable compositions of this invention include injectable salts such as the sodium salt of ampicillin, amoxycillin, carbenicillin and ticarcillin.

A preferred penicillin for inclusion in the injectably administrable compositions of this invention is sodium amoxycillin. A further preferred penicillin for inclusion in the injectably administrable compositions of this invention is sodium ampicillin.

Particularly suitable cephalosporins for inclusion in the compositions of this invention include cephaloridine, cephalexin, cephradine, cefazolin and cephalothin.

A particularly suitable cephalosporin for inclusion in the orally administrable compositions of this invention is cephalexin.

Particularly suitable cephalosporins for inclusions in the injectably administrable compositions of this invention include cefazolin and cephradine, generally as their pharmaceutically acceptable salt such as the sodium salt. Cephaloridine is also particularly suitable for inclusion in injectably administrable compositions.

The weight ratio between compound of this invention and penicillin or cephalosporin is generally from 10:1 to 1:10, for more usually from 5:1 to 1:5 and normally from 3:1 to 1:3.

The penicillin or cephalosporin is generally utilised in its conventionally administered amount.

In another aspect the present invention provides a process for the preparation of a compound of the formula (III) which process comprises the reaction of a compound of the formula (VI):

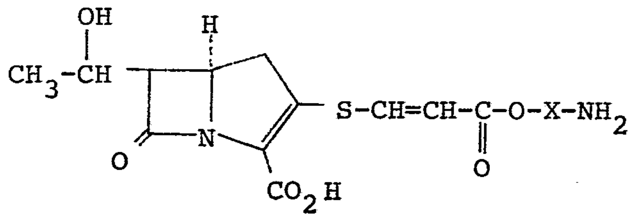

(VI)

or protected derivative thereof, wherein X is as defined in relation to a compound of the formula (III), with either a compound of the formula (VII) or (VIII):

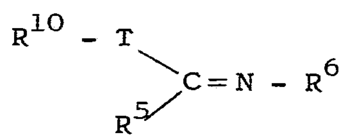

(VII)

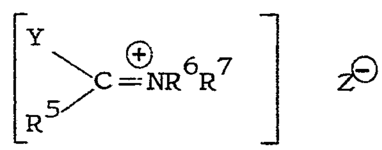

(VIII)

wherein $R^5$, $R^6$ and $R^7$ are as defined in relation to formula (III), $R^{10}$ is $C_{1-6}$ alkyl, T is oxygen or sulphur, Y is halo or $C_{1-6}$alkoxy, and Z is a salting-ion. Suitable protected derivatives include those that are cleavable under hydrolytic conditions, for example those conditions of the amidination reaction. Most suitably the C-8 hydroxy may be protected as its formate. In addition the formate derivatives are useful as antibacterial agents either in their own right or because they are believed to hydrolyse in-vivo to the compound of the formula (III).

The compound of the formula (VII) is preferably used in the form of its acid addition salt, for example as the hydrochloride.

Suitably $R^{10}$ is a methyl or ethyl group. Suitably T is an oxygen atom. Suitably also Y is $C_{1-6}$ alkoxy such as methoxy or ethoxy. Suitably Z is halo such as chloro, or is a tetrafluoroborate ion.

Reaction of the compound of the formula (VI) with either the compound of the formula (VII) or (VIII) is suitably performed in water, ether, dioxan, tetrahydrofuran, dimethylformamide, dichloromethane or mixtures of such solvents. Suitably the reaction is performed between $0^{o}C$ and ambient temperature. In aqueous media it has been found convenient to carry out the reaction at a basic pH, for example 7.5 to 10, preferably 8 to 9.

The following Examples illustrate the invention. In the Examples PNB means para-nitrobenzyl.

0072121



Example 1

(5R,6R)-3-[(Z)-2-Acetimidoylaminoethyloxycarbonyl-ethenylthio]-6-[(S)-1-hydroxyethyl]-7-oxo-1-azabicyclo[3.2.0]hept-2-ene-2-carboxylic acid

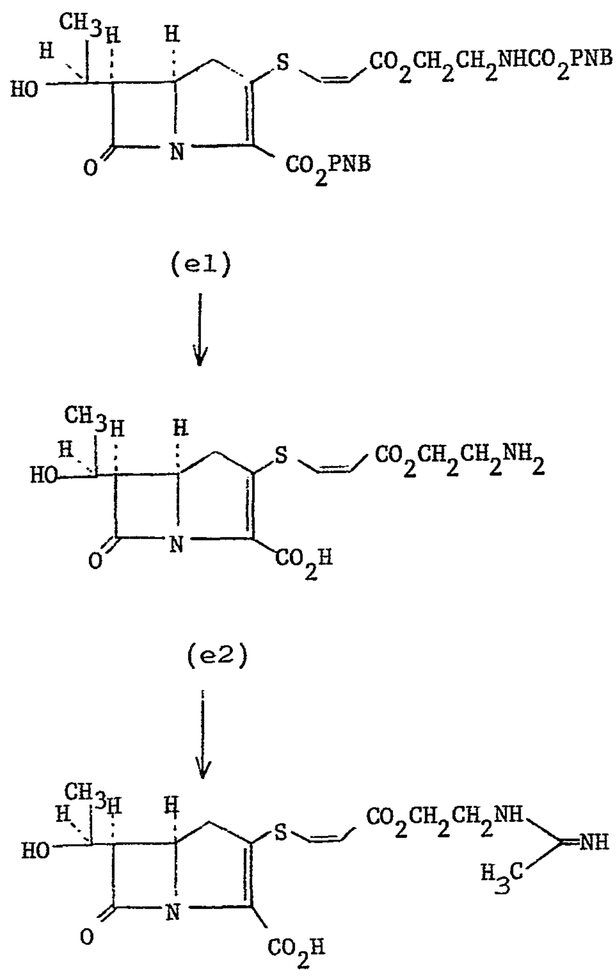

(e1)

(e2)

(e3)

A solution of p-nitrobenzyl (5R,6R)-3-[(Z)-2-p-nitrobenzyloxycarbonylaminoethyloxycarbonyl-ethenylthio]-6-[(S)-1-hydroxyethyl]-7-oxo-1-azabicyclo-[3.2.0]hept-2-ene-2-carboxylate (210 mg) in 1,4 dioxan (30 ml), water (9 ml) and 0.05 M pH 7.0 phosphate buffer

(9.5 ml) was shaken with hydrogen for 2 hours in the presence of 5% palladium on carbon catalyst (315 mg). The solution was filtered over Celite, washing well with water (50 ml). The filtrate was concentrated to approximately 40 ml and washed with ethyl acetate (3 x 50 ml). The aqueous solution, which was estimated to contain 72 mg of the amino acid (e2) based on $\varepsilon_m$ 17,000 at $\lambda_{max}$323 nm in the u.v. spectrum, was evaporated to small volume (10 ml) at reduced pressure. The solution was stirred at room temperature and the pH maintained at pH 8.5 by the addition of 1<u>N</u> sodium hydroxide solution whilst ethyl acetimidate hydrochloride (183 mg) was added over a period of 15 minutes. Stirring was continued at pH 8.5 for a further 45 minutes. The pH was then readjusted to 7.0 and the solution chromatographed over HP-20. The column was eluted with a gradient of 0 - 20% ethanol/water and the fractions monitored by u.v. Those fractions possessing an absorption at $\lambda_{max}$ 323 nm in the u.v. spectrum were combined and the resulting solution containing (5R,6R)-3-[(Z)-2-acetimidoyl-aminoethyloxycarbonylethenylthio]-6-[(S)-1-hydroxyethyl]-7-oxo-1-azabicyclo[3.2.0] hept-2-ene-2-carboxylic acid (e3) (37 mg) was concentrated to small volume. A portion of this solution was freeze-dried to yield a pale yellow fluffy solid, $\lambda_{max}(H_2O)$ 323 nm ($\varepsilon_m$ 10,311). $\nu_{max}$ (KBr) 3000 - 3500 (broad), 1750, 1685, 1638, 1570 $cm^{-1}$. H.P.L.C. (10% $CH_3CN$, 90% pH 4.7, 0.05M ammonium dihydrogen orthophosphate buffer; 2 ml. $min^{-1}$, 300.mm.$h^{-1}$, 320 nm) indicated that the product contained approximately 15% of the corresponding <u>E</u>-isomer.

Example 2

1) p - Nitrobenzyl (5R,6S)-3-[2-(2-p-nitrobenzyloxycarbonylaminoethoxycarbonyl) ethenylthio]-6-[(R)-1-formyloxyethyl]-7-oxo-1-azabicyclo [3.2.0] hept-2-ene-2-carboxylate.

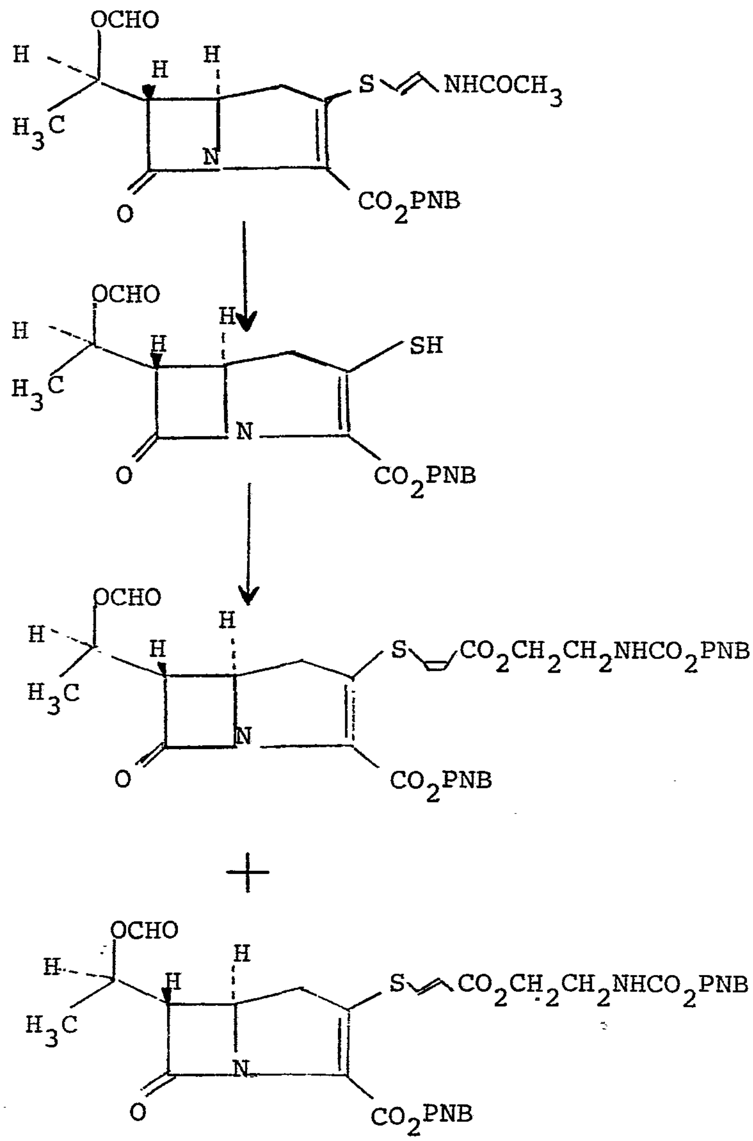

(e4)

(e5)

(e6)

(e7)

p - Nitrobenzyl (5R,6S)-3-(E-2-acetamidoethenylthio)-6-[(R)-1-formyloxyethyl]-7-oxo-1-azabicyclo [3.2.0] hept-2-ene-2-carboxylate (e4) (377mg) prepared by the methods of European Patent Application 81304618.2, was dissolved in aqueous 1,4 - dioxan (50ml) and stirred at room temperature for 5 minutes with N-bromoacetamide (109.5mg). Chloroform was added and the organic phase was washed with pH 7.0, 0.05M phosphate buffer, saturated sodium chloride solution and dried over anhydrous magnesium sulphate. Filtration and removal of the solvent at reduced pressure afforded the crude thiol (e5) as a pale yellow oil, $\nu_{max}$ ($CHCl_3$) 1781, 1723, 1610, 1560, 1525, 1355, 1340 cm.

The crude thiol (e5) was dissolved in dry dimethylformamide (10ml) and stirred at room temperature for 15 minutes with 2-(p-nitrobenzyloxycarbonylamino) ethyl propiolate (350mg) and anhydrous potassium carbonate (109.5mg). Ethyl acetate was added and the organic solution washed with water, saturated sodium chloride solution and dried over anhydrous magnesium sulphate. After filtration, the solvent was removed at reduced pressure to yield a yellow oil, which was chromatographed over silica gel (20gm). Elution with ethyl acetate afforded the crude product as a pale yellow foam (310mg). This foam was re-chromatographed over silica gel (15gm), eluting with chloroform. The resulting pale yellow oil, on trituration with diethyl ether afforded a pale yellow solid (155mg). This consisted of p-nitrobenzyl (5R,6S)-3-[2-(2-p-nitrobenzyloxycarbonylaminoethoxycarbonyl) ethenylthio]-6-[(R)-1-formyloxyethyl]-7-oxo-1-azabicyclo [3.2.0]hept-2-ene-2-carboxylate as a mixture of the Z - isomer (e6) and the E - isomer (e7) in the approximate ratio 9:1; $\lambda_{max}$ (EtoH) 334nm (εm17,746), 265nm (εm 18,579), $\nu_{max}$ ($CHBr_3$) 3400, 1781, 1720, 1605, 1520,

1345cm$^{-1}$, $\delta_H$($CDCl_3$) 1.47 (3H,d,$CH_3$CH), 3.24 (1H,dd,4-CHa), 3.4-3.6 (4H,m,$CH_2$NH + 6-CH + 4-$CH_b$), 4.30 (3H,m,$CO_2CH_2$ + 5-CH), 5.20 (2H,s,$CH_2$Ar), 5.22-5.58 (4H, q + m, $CH_2$Ar + 8-CH + NH), 6.00 (1H,d,J 10.5Hz, CH=CH ), 7.26 (1H,d,J 10.5Hz, CH=CH ), 7.49 (2H,d, aromatic protons), 7.66 (2H,d, aromatic protons), 8.07 (1H,S,CHO), 8.13-8.30 (4H,2xd, aromatic protons). The E-isomer shows inter alia δ6.12 (d,J 15Hz, CH=CH ), 7.77 (d,J 15Hz, CH=CH ).

<u>ii) (5R,6S)-3-[(Z)-2-Acetimidoylaminoethyloxycarbonylethenylthio]-6-[(R)-1-hydroxyethyl]-7-oxo-1-azabicyclo[3.2.0]hept-2-ene-2-carboxylic acid</u>.

(e6)
+
(e7)

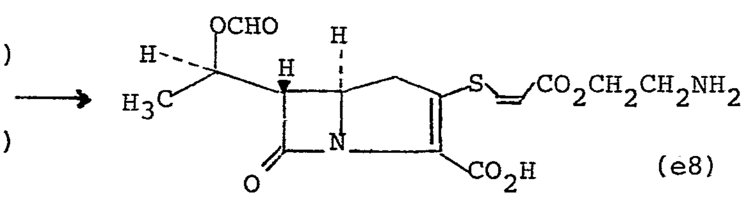

(e8)

(e9)

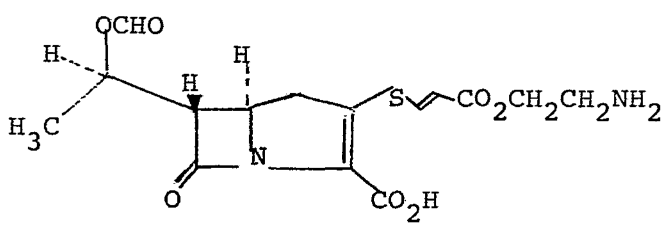

(e10)

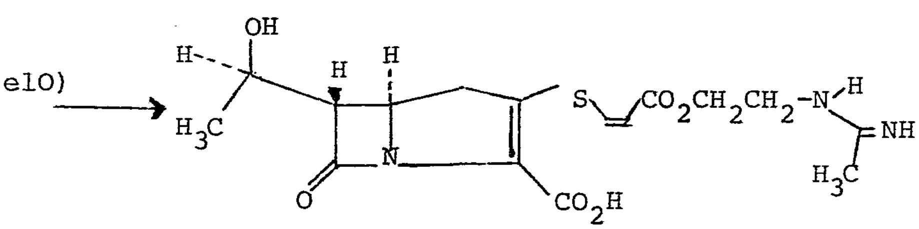

The mixtures of esters (e6) and (e7)(155mg) was dissolved in 1,4-dioxan (25ml), water (7.5ml) and pH 7.0, 0.05M phosphate buffer (7.2ml) and shaken for 2 hours with hydrogen in the presence of 5% palladium on carbon catalyst (240mg). The solution was filtered over celite, washing well with water (50ml). The filtrate was concentrated to approximately 40ml and washed with ethyl acetate (3 x 50ml). Theaqueous solution was estimated to contain 40mg (5R,6S)-3-[(Z)-2-aminoethyloxycarbonylethenylthio] -6-[(R)-1-formyloxyethyl]-7-oxo-1-azabicyclo [3.2.0]hept-2-ene-carboxylic acid (e8) based on εm 17,000 at $\lambda_{max}$ 323nm in the u.v. spectrum. H.P.L.C.(10%$CH_3CN$, 90% pH4.7, 0.05M, ammonium dihydrogen orthophosphate buffer; 2ml.min $\lambda_{max}$ 320nm; 300mm.$h^{-1}$) indicated that the solution contained approximately 14% of the E-isomer (e9).

The solution containing (e8) and (e9) was concentrated to 5ml at reduced pressure and cooled to $0^oC$. The pH was adjusted by the addition of 1N sodium hydroxide solution and ethyl acetimidate hydrochloride (267mg) was added over a period of 5 minutes with stirring. Stirring was continued at $0^oC$ for 1.5 hours. During this time the pH was kept between 8.5 and 9.0. The solution was allowed to reach room temperature and stirring continued at this pH for an additional hour, monitoring the reaction by u.v. and H.P.L.C. The pH was then adjusted to 7.0 and the solution chromatographed over Diaion HP-20, eluting with a gradient of 0-10% ethanol/water and monitoring the fractions by u.v. and H.P.L.C. Fractions 44-52, which were combined, contained pure (5R,6S)-3-[(Z)-2-acetimidoylaminoethyloxycarbonylethenylthio]-6-[(R)-1-hydroxyethyl]-7-oxo-1-azabicyclo[3.2.0] hept-2-ene-2-carboxylic acid (9.5mg). An aliquot of the solution was freeze-dried to yield a white fluffy solid, $\lambda_{max}$ ($H_2O$)

325nm (εm 10,760), $\nu_{max}$ (KBr) 3000-3500 (broad), 1760, 1686, 1638, 1568cm$^{-1}$, $\delta_H$($D_2O$) 1.28 (3H,d,J 6.5Hz,$CH_3$CH), 2.22(3H,S,$CH_3$- C=N), 3.22(1H,dd,4-$CH_a$), 3.32-3.50 (2H,m, 4-$CH_b$ + 6-CH), 3.60 (2H, broad res; $CH_2$N), 4.15-4.45 (4H, m,$CO_2CH_2$+5-CH+8-CH), 5.99 (1H,d,J10Hz, CH=CH ), 7.61 (1H,d,J 10Hz, CH=CH ).

Example 3

(5R,6R)-3-[(Z)-2-Formimidoylaminoethyloxycarbonylethenylthio]-6-[(S)-1-hydroxyethyl]-7-oxo-1-azabicyclo[3.2.0]hept-2-ene-2-carboxylic acid

(e1) (e2)

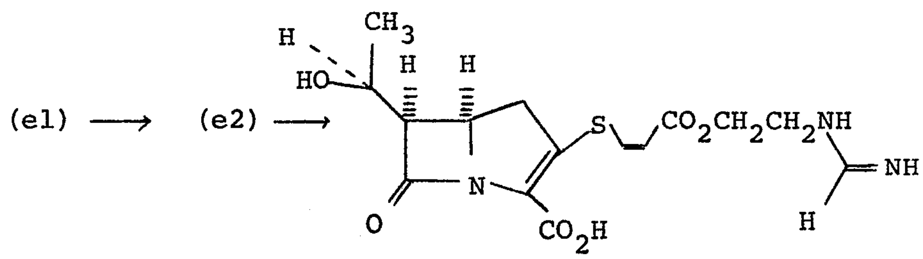

(e11)

The title compound was obtained as a white solid by reacting the amino-acid (e2) with ethyl formimidate hydrochloride (excess), following the procedure outlined in Example 1, $\lambda_{max}$ ($H_2O$) 324 nm ($\varepsilon$m 12748), $\nu_{max}$ (KBr) 3300 (broad), 1750, 1708, 1570 $cm^{-1}$; $\delta_H$ ($d_6$-DMSO) inter alia 7.71 (1H, d, vinylic 1<u>H</u>), 5.87 (1H, d, vinylic 1<u>H</u>), 1.21 (3H, d, $C\underline{H}_3CH$).

Example 4

(5R,6R)-3-{(Z)-2-N-[(2-pyridyl(imino)methyl]aminoethyl-oxycarbonylethenylthio}-6-[(S)-1-hydroxyethyl]-7-oxo-1-azabicyclo[3.2.0]hept-2-ene-2-carboxylic acid

(e1) ⟶ (e2) ⟶ 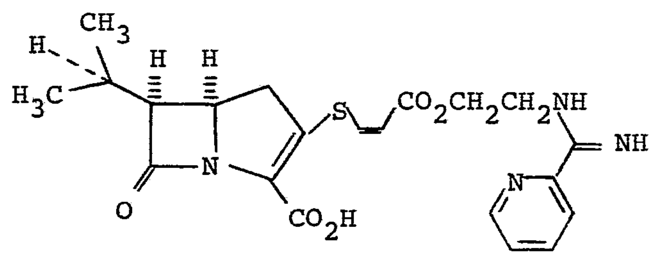

(e12)

The title compound was prepared by reaction of the amino acid (e2) with excess methyl picolinimidate, following the procedure outlined in Example 1. Chromatography of the reaction mixture over HP-20, eluting with a gradient of 0-20% ethanol/water afforded the pure product in aqueous solution, $\lambda_{max}$ ($H_2O$) 319 nm.

In a standard MIC test the compound of Examples 1 and 2 afforded the following data (μg/ml).

| Organisms | Ex.1 | Ex.2 |
|---|---|---|
| Citrobacter freundii E8 | 0.4 | 0.4 |
| Enterobacter cloacae N1 [T753] | 3.1 | 0.8 |
| Escherichia coli O111 [E8] | 0.2 | 0.2 |
| Escherichia coli JT39 [E96] | 0.2 | 0.2 |
| Escherichia coli ESS | ≤ 0.1 | 0.2 |
| Klebsiella aerogenes A [T767] | 0.2 | 0.2 |
| Proteus mirabilis 977 [13] | 0.4 | 0.8 |
| Proteus morganii 1580 | 1.6 | 1.6 |
| Proteus rettgeri WM16 | 1.6 | 0.8 |
| Proteus vulgaris W091 | 3.1 | 0.8 |
| Pseudomonas aeruginosa A [11] | 25 | 25 |
| Salmonella typhimurium CT10 | 0.2 | 0.2 |
| Serratia marcescens US20 | - | - |
| Shigella sonnei MB11967 | 0.2 | 0.2 |
| Bacillus subtilis A | ≤ 0.1 | ≤ 0.1 |
| Staphylococcus aureus Oxford | ≤ 0.1 | ≤ 0.1 |
| Staphylococcus aureus Russell MB9 | ≤ 0.1 | ≤ 0.1 |
| Staphylococcus aureus 1517 | 0.8 | 1.6 |
| Streptocuccus faecalis I | ≤ 0.1 | 0.2 |
| Streptococcus pneumoniae CN33 | ≤ 0.1 | - |
| Streptocuccus pyogenes CN10 | ≤ 0.1 | - |
| Escherichia coli JT410 | 0.2 | 0.2 |
| Escherichia coli JT20 | 3.1 | 0.2 |
| Klebsiella aerogenes Ba95 | 3.1 | 0.4 |
| Pseudomonas Dalgleish | 25 | 25 |

A.



CLAIMS:

1. An amidine derivative of an amine of formula (II):

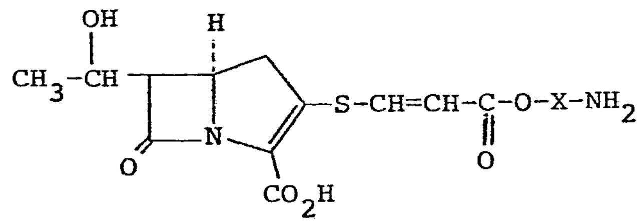

(II)

or a pharmaceutically acceptable salt or in vivo hydrolysable ester thereof where X represents a $C_{1-6}$ alkanediyl moiety.

2. A compound as claimed in claim 1 which is of formula (III):

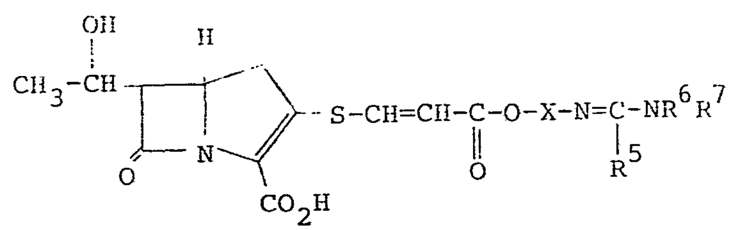

(III)

or a pharmaceutically acceptable salt or in vivo hydrolysable ester thereof, wherein X is a $C_{1-6}$alkanediyl moiety, and $R^5$, $R^6$ and $R^7$ are independently selected from hydrogen, a heterocyclic group, a hydrocarbon group up to 18 carbon atoms; or $R^6$ and $R^7$ together with the nitrogen atom to which they are attached form a $C_{3-10}$ cycloalkyl ring; any of the above $R^5$, $R^6$ and $R^7$ groups being optionally substituted.

3. A compound as claimed in claim 2 wherein X represents an ethylene moiety.

4. A compound as claimed in any one of claims 1 to 3 wherein $R^6$ is hydrogen.

5. A compound as claimed in claim 4 wherein $R^7$ is hydrogen.

6. A compound as claimed in any one of claims 1 to 5 wherein $R^5$ represents hydrogen, $C_{1-6}$ alkyl or heterocycyl having 4-6 ring atoms.

7. A compound selected from:

3-[(Z)-2-Acetimidoylaminoethyloxycarbonylethenylthio]-6-[(S)-1-hydroxyethyl]-7-oxo-1-azabicyclo-[3.2.0]hept-2-ene-2-carboxylic acid;

3-[(Z)-2-Formimidoylaminoethyloxycarbonylethenylthio]-6-[(S)-1-hydroxyethyl]-7-oxo-1-azabicyclo-[3.2.0]hept-2-ene-2-carboxylic acid;

3-{(Z)-2-N-[(2-pyridyl)(imino)methyl]aminoethyloxycarbonylethenylthio}-6-[(S)-1-hydroxyethyl]-7-oxo-1-azabicyclo[3.2.0]hept-2-ene-2-carboxylic acid.

8. A process for the preparation of a compound as claimed in claim 2, which process comprises the reaction of a compound of the formula (VI):

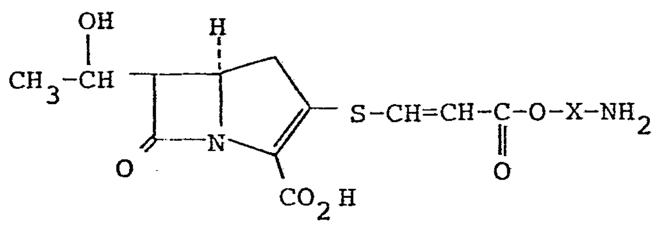

(VI)

or protected derivative thereof,
wherein X is as defined in relation to a compound of the formula (III), with either a compound of the formula (VII) or (VIII):

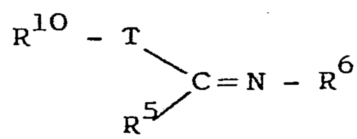

(VII)

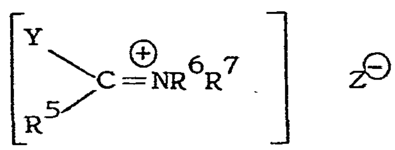

(VIII)

wherein $R^5$, $R^6$ and $R^7$ are as defined in relation to formula (III), $R^{10}$ is $C_{1-6}$ alkyl, T is oxygen or sulphur, Y is halo or $C_{1-6}$alkoxy, and Z is a salting-ion.

9. A pharmaceutical composition which comprises a compound as claimed in any one of claims 1 to 7 together with a pharmaceutically acceptable carrier.

10. A compound as claimed in any one of claims 1 to 7 for use in the treatment of bacterial infections.

0072121

European Patent Office

# EUROPEAN SEARCH REPORT

Application number

EP 82 30 3859

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | EP-A-0 033 209 (BEECHAM)<br>* claims * | 1,2,9,<br>10 | C 07 D 487/04<br>A 61 K 31/40 //<br>(C 07 D 487/04<br>C 07 D 209/00<br>C 07 D 205/00 ) |
| D,A | EP-A-0 024 832 (BEECHAM)<br>* pages 47-49; examples 18-20; claims 3-6, 10,19 * | | |
| | | | TECHNICAL FIELDS SEARCHED (Int. Cl. 3)<br>C 07 D 487/00<br>A 61 K 31/00 |

The present search report has been drawn up for all claims

| Place of search | Date of completion of the search | Examiner |
|---|---|---|
| THE HAGUE | 28-10-1982 | CHOULY J. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503. 03.82